Europäisches Patentamt

(19) European Patent Office

Office européen des brevets

(11) Veröffentlichungsnummer: **0 131 759**
**B1**

(12) **EUROPÄISCHE PATENTSCHRIFT**

(45) Veröffentlichungstag der Patentschrift:
27.08.86

(51) Int. Cl.⁴: **B 65 D 30/02**, A 01 M 1/20,
A 01 N 25/34

(21) Anmeldenummer: 84106747.3

(22) Anmeldetag: 13.06.84

(54) Beutel für Schädlingsbekämpfungsmittel.

(30) Priorität: 18.07.83 DE 3325826

(43) Veröffentlichungstag der Anmeldung:
23.01.85 Patentblatt 85/4

(45) Bekanntmachung des Hinweises auf die Patenterteilung:
27.08.86 Patentblatt 86/35

(84) Benannte Vertragsstaaten:
AT CH DE FR GB LI NL

(56) Entgegenhaltungen:
WO - A - 80/00119
DD - A - 149 791
DE - A - 2 454 172

(73) Patentinhaber: DR. WERNER FREYBERG, CHEMISCHE
FABRIK, DELITIA NACHF., Bergstrasse,
D-6941 Laudenbach (DE)

(72) Erfinder: Friemel, Wolfgang, Dr., Giessener Strasse 4,
D-6148 Heppenheim (DE)
Erfinder: Ehret, Reiner, Forlenweg 14, D-6940 Weinheim
(DE)

(74) Vertreter: Weinhold, Peter, Dr. et al, Patentanwälte Dr. V.
Schmied-Kowarzik Dipl.-Ing. G. Dannenberg Dr. P.
Weinhold Dr. D. Gudel Dipl.-Ing. S. Schubert Dr. P. Barz
Siegfriedstrasse 8, D-8000 München 40 (DE)

**Beschreibung**

Die Erfindung betrifft einen Beutel, der zur Aufnahme eines gasentwickelnden Schädlingsbekämpfungsmittels dienen kann (siehe, z.B., WO-A-80/00119).

Es sind Schädlingsbekämpfungsmittel fester Konsistenz bekannt, die an feuchter Luft langsam gasförmige Bestandteile abgeben. Solche Schädlingsbekämpfungsmittel können hydroliserbare Erdalkali- und/oder Erdmetallphosphide enthalten, die unter dem Einfluss von Luft bzw. Lagergutfeuchtigkeit Phosphin abspalten. Derartige Schädlingsbekämpfungsmittel werden beispielsweise zur Schädlingsbekämpfung in Lagerhäusern bzw. Silos für Getreide, Tabak oder ähnliche Nahrungs- bzw. Genussmittel eingesetzt. Das Schädlingsbekämpfungsmittel dient dabei der Vernichtung von tierischen Organismen, wie z.B. Käfern, Motten oder sonstigen Insekten sowie Würmern und Nagetieren, wie Ratten und Mäusen. Es ist bekannt, hierfür derartige Schädlingsbekämpfungsmittel in Papierbeutel zu füllen und in das Lagergut einzubringen. Das Schädlingsbekämpfungsmittel sollte dann in dem Papierbeutel langsam zerfallen und seine Schädlings-bekämpfenden, gasförmigen Bestandteile durch die Wände des Papierbeutels nach aussen entlassen. Die staubförmigen Rückstände des Schädlingsbekämpfungsmittels sollen dabei von dem Papierbeutel zurückgehalten werden. An das hierfür verwendete Papier werden besondere Anforderungen bezüglich Festigkeit, Gasdurchlässigkeit und wasserabweisender Ausrüstung gestellt.

Solche Papiere können aber nur sehr bedingt auf automatischen Anlagen (wie z.B. Schlauchbeutelmaschinen, Siegelrandbeutelmaschinen etc.) verarbeitet werden. Sie werden zum überwiegenden Teil durch Nähen verschlossen, was einen hohen Arbeitsaufwand erfordert. Auch kann es vorkommen, dass die Nähte aufgehen und der giftige Inhalt in das zu behandelnde Nahrungsmittel gelangt.

Des weiteren ist ein Beutel zur Aufnahme von Phosphin entwickelnden Schädlingsbekämpfungsmitteln bekannt, der aus einem gasdurchlässigen Vliesstoff besteht und eine bestimmte Maschenweite aufweist. Als Material für den Vliesstoff sind Vliese auf der Grundlage von Zellulose oder aus bestimmten synthetischen Polymeren genannt. Es ist auch angegeben, dass die Herstellung des Beutels aus einer Vliesstoffbahn u.a. durch Verschweissen unter Anwendung von Wärme und/oder Ultraschall vorgenommen werden kann. Dieses ist insofern sehr zweckmässig, als die Herstellung einer Schweissnaht weniger arbeitsaufwendig ist als die Verbindung von Vliesstoffbahnen bzw. verschiedenen Seiten einer Vliesstoffbahn durch Kleben oder Nähen.

Es hat sich jedoch herausgestellt, dass es wünschenswert ist, die Verschweissbarkeit der für die bekannten Beutel verwendeten Vliesmaterialien zu verbessern. Das gilt sowohl hinsichtlich der Erzeugung einer Schweissnaht in der Praxis als auch hinsichtlich deren Festigkeit und Zuverlässigkeit bei der Verwendung des mit Schädlingsbekämpfungsmittel gefüllten Beutels.

Der Erfindung liegt die Aufgabe zugrunde, einen Beutel aus einem Material zu schaffen, das eine für die Anwendung erforderliche Gasdurchlässigkeit aufweist, jedoch die staubförmigen Rückstände des zerfallenen Schädlingsbekämpfungsmittels zurückhält, zusätzlich eine hohe Haltbarkeit besitzt sowie hervorragend geeignet ist, durch Verschweissung durch Wärme und/oder Ultraschall, insbesondere Wärme, zu einem zweckmässigen Beutel verarbeitet zu werden.

Diese Aufgabe wird erfindungsgemäss dadurch gelöst, dass der Beutel aus einem speziellen Vliesstoff in an sich bekannter Weise gefertigt wird.

Gegenstand der Erfindung ist also ein Beutel für Schädlingsbekämpfungsmittel, zumindest teilweise bestehend aus einem Gas- und Wasserdampf-durchlässigen, wasserfreien Vliesstoff, der dadurch gekennzeichnet ist, dass der Vliesstoff ein Mehrkomponentenvliesstoff ist, welcher mindestens ein faserbildendes Material mit einem Schmelz- bzw. Erweichungspunkt über 165° C und ein zweites Material mit thermoplastischen Eigenschaften und einem Schmelz- bzw. Erweichungspunkt unter 145° C umfasst.

Nach bevorzugten Ausführungsformen der vorliegenden Erfindung besitzt das faserbildende Material einen Schmelz- bzw. Erweichungspunkt zwischen 180 und 235° C, während das zweite Material vorzugsweise einen Schmelz- bzw. Erweichungspunkt zwischen 80 und 120° C aufweist.

Sofern kein eindeutiger Schmelz- bzw. Erweichungspunkt der betreffenden Materialien vorliegt, gelten die obigen bzw. in den Ansprüchen genannten Angaben sinngemäss für einen entsprechenden Schmelz- bzw. Erweichungsbereich.

Es ist also ein wesentliches Merkmal der vorliegenden Erfindung, dass zur Herstellung des erfindungsgemässen Beutels ein Vliesstoff aus mehreren Komponenten verwendet wird. Eine Komponente davon ist ein zur Faserbildung geeignetes Material mit relativ hohem Schmelz- bzw. Erweichungspunkt. Derartige Materialien, die zur Herstellung von Vliesstoffen geeignet sind, sind in grösserer Zahl bereits bekannt und zum Teil auch bereits im Handel erhältlich. Zu speziellen Beispielen für diese Komponente zählen Polyamide, Polyester, Polyacrylverbindungen, insbesondere Polyacrylamid sowie Glasfasern. Für schnellaufende Vorrichtungen kann als höherschmelzende Komponente auch Polypropylen mit relativ hohem Erweichungspunkt eingesetzt werden, beispielsweise in Kombination mit Polyäthylen als niedrigschmelzender Komponente.

Das als weitere Komponente verwendete zweite Material mit thermoplastischen Eigenschaften kann in verschiedener Form vorliegen. Nach einer speziellen Ausführungsform bildet diese Komponente ebenfalls Fasern, welche z.B. eine Länge von 0,5 bis 1 mm haben können.

Es ist jedoch auch möglich, dass die zweite Komponente auf die Fasern des Materials mit dem höheren Schmelz- bzw. Erweichungspunkt, ins-

3      **0 131 759**      4

besondere in den Bereichen des Vliesmaterials, wo eine Schweiss- bzw. Versiegelungsnaht vorgesehen ist, in an sich bekannter Weise aufgebracht wird. Dieses ist beispielsweise durch das folgende Besinterungsverfahren möglich:

Das Material, welches für die Besinterung verwendet wird, wird zunächst in einer tiefgekühlten, geeigneten Mahlanlage zu einem feinen Pulver vermahlen. Dieses Pulver wird dann über Dosierwalzen auf das vorbeilaufende Vies möglichst gleichmässig aufgebracht. Dann läuft das Vlies an Ultrarot-Strahlern geeigneter Temperatur vorbei, wobei die Beschichtungsteilchen anschmelzen. Gegebenenfalls kann direkt anschliessend auch noch eine Kalandrierstufe vorgesehen sein, wodurch die angeschmolzenen Teilchen noch etwas breit gewalzt und noch besser fixiert werden.

Eine andere Methode des Auftrags der zweiten Komponente auf das faserbildende Material besteht in einem Aufdrucken auf der Innenseite der Vliesstofflage. Die Aufbringung des zweiten Materials kann jedoch auch in Form einer Lösung oder Suspension eines entsprechenden thermoplastischen Materials erfolgen.

Das zweite Material bzw. ein weiteres thermoplastisches Material kann ganz oder überwiegend dazu dienen, die Bildung der Schweissnähte zu ermöglichen oder zu verbessern oder die Gasdurchlässigkeit und das Zurückhaltevermögen des Vliesstoffes gegenüber feinem Pulver in der gewünschten Weise zu verändern. Das Aufbringen der entsprechenden Materialien kann also über den gesamten Oberflächenbereich des Vliesstoffes oder nur auf bestimmte Teile von diesem, insbesondere an den Stellen, wo die Schweissnähte vorgesehen sind, erfolgen.

Aus Sicherheitsgründen sollte die Breite beispielsweise des Aufdrucks auf diejenigen Stellen des Vliesmaterials, auf denen eine Schweissnaht vorgesehen ist, mindestens etwa 1 cm, vorzugsweise von 1 bis 3 cm, insbesondere 1 bis 2 cm betragen.

Als niedriger schmelzende Komponente des Vliesstoffs kommen insbesondere Polyäthylen und entsprechende Polypropylenmaterialien oder auch Copolymere von Äthylen, Propylen oder Butylen mit Vinylacetat in Frage. Je nach dem vorliegenden speziellen Fall können vom Fachmann geeignete Materialien ausgewählt werden.

Bei der speziellen Ausführungsform der Erfindung, bei der auch die zweite Komponente in Form von separaten Fasern vorliegt, ist die Faserlänge der höherschmelzenden Komponente vorzugsweise grösser als diejenige der niedriger schmelzenden Komponente, wobei erstere zwischen 5 und 20 mm betragen kann, während letztere vorzugsweise zwischen 0,5 bis 1 mm liegen kann.

Bei diesen erfindungsgemässen Beuteln werden dabei die Zwischenräume zwischen den langen Fasern aus der höher schmelzenden Komponente durch die in kürzeren Fasern vorliegende niedriger schmelzende Komponente ausgefüllt, was selbstverständlich die gewünschte Staubdichtigkeit der erfindungsgemässen Beutel wesentlich verbessert.

Das Mengenverhältnis zwischen den beiden genannten Komponenten ist innerhalb relativ weiter Grenzen variierbar. So werden nach einer speziellen Ausführungsform der Erfindung Anteile an der höher schmelzenden Komponente von 50 bis 70 Gew.-% (bezogen auf die Gesamtmenge an höher schmelzender Komponente und niedriger schmelzender Komponente) mit gutem Erfolg verwendet.

Als Material für den zur Herstellung des erfindungsgemässen Beutels verwendbaren Vliesstoff können aber auch geeignete Bi-Komponentenfasern, bei denen es sich um Kern-Mantel-Fasern handelt, verwendet werden. Dabei bildet das faserbildende Material den Kern, während das zweite Material den Mantel bildet. Ein Beispiel derartiger Bi-Komponentenfasern sind solche, bei denen der Kern aus Polyamid 6.6 und der Mantel aus Polyamid 6 besteht.

Die Porosität des erfindungsgemäss verwendeten Vliesstoffs ist derart, dass zwar Gase noch ohne weiteres hindurchtreten können, jedoch Stäube, wie sie bei der Hydrolyse der Schädlingsbekämpfungsmittel auftreten, am Durchgang gehindert werden. Es sind Porengrössen des Vliesstoffes von mehr als 2, insbesondere von 5 bis 15 µm, vorzugsweise zwischen 10 und 12 µm gut geeignet. Bei diesen Werten treten praktisch keinerlei Rückstände der Schädlingsbekämpfungsmittel aus dem erfindungsgemässen Beutel aus.

Die Herstellung der erfindungsgemäss zur Beutelherstellung eingesetzten Vliesstoffe kann entsprechend völlig bekannter Verfahren durchgeführt werden. Aus diesem Grunde ist es zweifellos ausreichend, an dieser Stelle nur einige wesentliche Gesichtspunkte anzugeben.

Insbesondere die erfindungsgemäss verwendeten Vliesstoffe aus verschiedenen Fasern können grundsätzlich wie folgt verarbeitet werden und aus folgenden Materialien bestehen:

Aus Krempelvliesen, zu deren Herstellung das Fasermaterial auf einer Krempel entweder in Laufrichtung parallel oder über einen Kreuzleger zickzack-förmig auf ein quer zur Krempel laufendes Band gelegt wird,

aus aerodynamischen Vliesen, zu deren Herstellung Einzelfasern mit einem Luftstrom auf ein Siebband oder auf eine Siebtrommel geblasen werden, wobei ein Wirrvlies entsteht,

oder aus Spinnvliesen, zu deren Herstellung ein geeignetes Polymer in Granulatform aufgeschmolzen und durch eine schwenkbar angeordnete Spinndüse zu feinen Endlosfäden von einem heissen Luftstrom abgezogen und möglichst stark verstreckt wird.

Erfindungsgemäss bevorzugt werden jedoch hydrodynamisch hergestellte Vliese, die nach einer bevorzugten Ausführungsform wie folgt hergestellt werden können:

Es werden sowohl die Fasern der höher schmelzenden Komponente als auch diejenigen der niedriger schmelzenden Komponente in geeigneten Gefässen (Bütten) in einem relativ hohen Flottenverhältnis von bis zu 5000 Teilen Wasser zu 1 Teil Fasern suspendiert. Um Unregelmässigkeiten bei

3

dem hergestellten Vliesstoff zu vermeiden, ist es dabei zweckmässig, eine möglichst vollständige Auflösung der Faserbündel bis zur Einzelfaser vorzunehmen, wobei die Verwendung eines oberflächenaktiven Mittels in dem wässerigen Medium vorteilhaft ist. Aufgrund der verhältnismässig grossen Wassermengen ist die Verwendung eines Schrägsiebs sowie von Saugkästen unter dem Schrägsieb vorteilhaft.

Nach der Bildung des Faservlieses wird vorzugsweise eine Verfestigung von diesem zum Vliesstoff und gegebenenfalls eine Veredelung des Vliesstoffes durchgeführt.

Für die Verfestigung gibt es grundsätzlich drei verschiedene Methoden, nämlich die mechanische Verfestigung (beispielsweise durch Nadelfilztechnik oder mit Hilfe von Wasserstrahlen), die chemische Verfestigung sowie die thermische Verfestigung. Von diesen drei Methoden kommt für hydrodynamisch gelegte Vliesen in erster Linie die chemische Verfestigung in Betracht, wobei man die Fasern des Vliesstoffs durch ein Bindemittel miteinander verklebt. Ein derartiges Bindemittel wird vorzugsweise in einer Menge von 15 bis 50 Gew.-%, vorzugsweise von 20 bis 30 Gew.-% (bezogen auf das Endgewicht des Vliesstoffes), angewendet. Sehr gut geeignet sind die an sich bekannten Bindemittel auf Kautschukbasis oder synthetische Harze, wie Polyacrylsäureester, Polyvinylchlorid, Polyvinylacetat oder Polyurethane. Selbstverständlich können auch geeignete Mischpolymerisate, wie beispielsweise Copolymere von Vinylacetat mit Acrylsäure- oder Methacrylsäureestern oder mit Polyolefinen, z.B. Polyäthylen, mit gutem Erfolg verwendet werden. Es werden zweckmässig entsprechende Kunststoffdispersionen eingesetzt.

Das verwendete Bindemittel kann zumindest zum Teil der Fasersuspension zugegeben und/oder auf die Vliese auf verschiedene Weise aufgebracht werden, wobei beispielsweise folgende Verfahren angewendet werden können:

Eine Imprägnierung, bei der das Vlies durch eine Bindemittelflotte geführt, getränkt zwischen zwei Walzen abgequetscht und anschliessend getrocknet wird.

Ein Sprühverfahren, bei dem das Faservlies auf einem Transportsieb unter Sprühdüsen, welche sich gegebenenfalls hin und her bewegen, besprüht wird,

das Aufbringen eines Schaumes, bei dem das Vlies mit aufgeschäumtem Bindemittel durchtränkt, danach aber vorzugsweise nicht abgepresst wird, das Pflatschverfahren, bei dem das Bindemittel über eine rotierende Walze am Vlies abgestreift wird, wobei der Vliesstoff nur eine einseitige Bindung erhält, und schliesslich das Aufbringen des Bindemittels durch Druckwalzen.

Nach Aufbringen des Bindemittels, aber auch an jeder anderen Stelle des Verfahrens, bei der eine Trocknung des Vliesstoffes notwendig oder wünschenswert ist, kann diese beispielsweise mit Infrarotstrahlern oder vorzugsweise mit Durchlufttrocknern durchgeführt werden.

Bei der thermischen Verfestigungsmethode werden schliesslich die thermoplastischen Eigenschaften der verwendeten synthetischen Fasern zur Schmelzverklebung ausgenutzt, wobei eine entsprechende kurzzeitige Hitzeeinwirkung durchgeführt wird.

Als Veredelungsverfahren kommen beispielsweise ähnliche Verfahren in Frage, wie sie bei der konventionellen Textilfertigung üblich sind, wie Kalandrieren, Bedrucken, Färben, Behandlung mit flammfestmachenden Ausrüstungen etc. Diese Veredelung kann in analoger Weise und unter Verwendung von ähnlichen Materialien erfolgen, wie es bekannt ist. Dabei soll ein eventueller Farbstoffgehalt 2 bis 3 Gew.-% der Gesamtzusammensetzung nicht überschreiten.

Ein Farbstoff kann auch in das faserbildende Material, in das zweite Material oder gegebenenfalls in das Bindemittel eingebracht werden. Vorzugsweise können die Beutel dadurch derart gefärbt sein, dass diese bei der Verwendung einen deutlichen Hinweis auf die Herstellungsstätte und – aufgrund früherer Markterfahrung – einen Hinweis auf die Inhaltsstoffe erhalten. So ist der Verbraucher an grüne Papierbeutel gewöhnt, so dass es sich anbietet, vorzugsweise auch die erfindungsgemässen Beutel mit einer grünen Ausstattung zu versehen. Selbstverständlich ist es auch vorteilhaft, die Bezeichnung des Schädlingsbekämpfungsmittels, Gebrauchsanweisungen und Warnungen vor unsachgemässem Gebrauch der Schädlingsbekämpfungsmittel etc. auf die erfindungsgemässen Beutel aufzudrucken.

Das Flächengewicht des hergestellten Vliesstoffs liegt vorzugsweise zwischen 50 und 120 g/m², wobei die obere Grenze u.a. von dem Verlust der notwendigen Flexibilität des Materials bestimmt wird. Nach einer speziellen Ausführungsform der Erfindung beträgt das Flächengewicht des Vliesstoffes etwa 70 g/m².

Bei der speziellen Ausführungsform der erfindungsgemässen Beutel, bei der eine Beschichtung des Vliesstoffes oder der Komponente mit höherem Schmelz- bzw. Erweichungspunkt vorgesehen ist, wird diese mit einem geeigneten Kunststoff, beispielsweise mit Polyäthylen oder einem Äthylen/Vinylacetat-Copolymer, vorgenommen. Eine solche Beschichtung kann z.B. in einer Menge zwischen 15 und 50 g/m², insbesondere zwischen 20 und 30 g/m², vorgenommen werden. Die für die Beschichtung zu verwendenden Verfahren sind bekannt (z.B. Besinterung).

Zur Herstellung der erfindungsgemässen Beutel kann Vliesstoff von einer Vliesstoffbahn entsprechend gefaltet und an den Längsseiten verschweisst werden. In die verbleibende Öffnung lässt sich nun das feste, vorzugsweise granulierte Schädlingsbekämpfungsmittel einfüllen. Danach wird auch noch die verbliebene Öffnung verschlossen. Eine andere Möglichkeit sieht vor, den Vliesstoff auf einer automatischen Schlauchbeutelmaschine in an sich bekannter Weise zu fertigen und gleichzeitig zu befüllen. Selbstverständlich ist es allgemein besonders zweckmässig, zur Herstellung und/oder zum Füllen der erfindungsgemässen Beutel Vorrichtungen zu verwenden, die in verschiedenen Formen bereits auf dem Markt sind. So sind beispielsweise geeignete automatische Vorrichtungen auf der Grundlage einer Wärmeverschweissung oder Verschweissung mit Ultraschall erhältlich. Zweckmässige Abmessungen des erfindungsgemässen Beutels richten sich selbstverständlich nach der gewünschten Menge des aufzunehmenden Schädlingsbekämpfungsmittels.

Nach dem Verschliessen des erfindungsgemässen Beutels ist das Schädlingsbekämpfungsmittel vollkommen eingeschlossen und die bei dessen Zersetzung gebildeten Stäube können aus dem Beutel nicht mehr austreten. Somit kann der Beutel mit dem Schädlingsbekämpfungsmittel direkt zu den zu begasenden Lebensmitteln gegeben werden.

Die Herstellung eines Siegelrandbeutels kann wie folgt durchgeführt werden:

Eine entsprechend breite Bahn des Vliesstoffes wird zunächst einmal in zwei gleichbreite Bahnen zerschnitten, die dann übereinandergelegt werden, so dass sie sich beim Schweissvorgang gegenüberstehen. Dann wird mit zwei vertikalen und einem horizontalen Schweisswerkzeug eine U-förmige Schweissnaht gebildet. Zu diesem Vorgang kann auch ein einziges U-förmiges Schweisswerkzeug benutzt werden. In die so hergestellte, nach oben offene Tasche wird dann das Schädlingsbekämpfungspräparat gefüllt. Danach bewegen sich die Vliesbahnen einen Takt weiter nach unten, und es wird wiederum eine U-förmige Schweissnaht erzeugt. Mit dem unteren Balken von dieser wird gleichzeitig der vorher gefüllte Beutel verschlossen.

Bei einer grösseren Vorrichtung können beispielsweise zwei Schweisswerkzeuge nebeneinander angeordnet sein, so dass gleichzeitig zwei nebeneinanderstehende Beutel entstehen, die z.B. in der Mitte zusammenhängen. Wenn man z.B. die Vliesstoffbahn nach 50 Schweiss- und Füllvorgängen (jeweils doppelt) horizontal durchschneidet, erhält man eine Anordnung mit 100 Beuteln in zwei Reihen nebeneinander. Man kann aber auch andere Beutelkombinationen wählen, wie beispielsweise zwei Zehnerketten, wobei man den Schneidevorgang vertikal nach jedem Takt und horizontal nach zehn Takten durchführt, oder nach jedem Takt sowohl horizontal als auch vertikal schneidet, wobei man jeweils zwei Einzelbeutel erhält.

Gegenstand der Erfindung sind auch Mehrfachbeutel bzw. Beutel mit mehreren Kammern. Dabei dienen Zwischennähte, welche beispielsweise durch Wärme oder Ultraschall gebildet werden, zur Unterteilung in Kammern bzw. Segmente. Doppelbeutel können beispielsweise eine gemeinsame Quernaht am Ende der Längsseiten besitzen. Nach einer speziellen Ausführungsform der vorliegenden Erfindung ist die Mehrzahl der erfindungsgemässen Beutel mit einem Flächengebilde von grösserer Abmessung als die einzelnen Beutel verbunden. Die Verbindung kann in jeder geeigneten Form, beispielsweise durch Verkleben oder auch durch Verschweissung unter dem Einfluss von Wärme bzw. Ultraschall erfolgen. Das Flächengebilde kann aus einem flexiblen Material bestehen und z.B. als eine Folie, in Form eines längeren Bandes, vorliegen, beispielsweise aus einem geeigneten Kunststoffmaterial, wie einem Polyolefin und dessen Mischpolymeren, Polyestern, Gemischen aus Polyolefin(en) und Polyester(n) sowie modifiziertes, nicht hygroskopisches Zellulosematerial. Es besteht dann die Möglichkeit einer sehr raschen und einfachen Auslegung der Beutel. Es ist lediglich erforderlich, ein Ende dieses Bandes an einer erhöhten Stelle zu fixieren und dieses Band in geeigneter Form abrollen zu lassen, wonach dann jeder Beutel der Atmosphäre des Silos, Lagerhauses, Transportmittels etc. ausgesetzt ist. In diesem Zusammenhang sei auf die PCT-Anmeldung WO-A-80/00119 verwiesen.

Sofern das Band aus dem gleichen oder einem ähnlichen Vliesstoff wie der erfindungsgemässe Beutel besteht, liegt auch die Möglichkeit vor, dass dieses einen integralen Teil des Beutels darstellt, indem es jeweils eine oder beide Beutelwände bildet. In einem speziellen Fall kann für das Band und die einzelnen Beutel das gleiche Vliesstoffmaterial verwendet werden.

Um das Beutelmaterial besser wasserabstossend zu machen, können die Fasern, die Vliesstoffbahnen und/oder die fertigen Beutel mit einem geeigneten hydrophobierenden Mittel behandelt, beispielsweise imprägniert, werden.

Ein weiterer Gegenstand der Erfindung ist die Verwendung eines Beutels zur Aufnahme ausgasungsfähiger Schädlingsbekämpfungsmittel, insbesondere einer Phosphin entwickelnden Zusammensetzung auf der Basis von Erdalkali- und/oder Erdmetallphosphiden, insbesondere Calciumphosphid, Magnesiumphosphid, vorzugsweise Aluminiumphosphid.

Die erfindungsgemässen Beutel, ob sie nun als Einzelbeutel, Mehrfachbeutel oder Flächengebilde, z.B. Band, vorliegen, dienen in der praktischen Anwendung als kombinierte Verpackungs- und Anwendungsvorrichtung für das darin enthaltene Begasungsmittel. Sie werden mit vorzugsweise exakt bemessenen Portionen des Begasungsmittels gefüllt, zugeschweisst und normalerweise anschliessend für die weitere Lagerung und den Versand einzeln oder zu mehreren in eine gas- und feuchtigkeitsdichte Verpackung gegeben, z.B. in eine Aluminiumfolienverpackung oder eine Dose z.B. aus Blech oder ggf. Metallfolien-kaschiertem Kunststoff.

Bei der Anwendung der Beutel oder Bänder ist es z.B. manchmal erwünscht, wenn diese mit einer Befestigungs- oder Aufhängemöglichkeit, z.B. einer Schnur, versehen sind. Es ist deshalb vorteilhaft, dass die erfindungsgemäss vorgesehenen Schweissnähte einen Bereich bilden, der sich wegen seiner Festigkeit und Verdichtung durch den Schweissvorgang besonders gut zur Anbringung von Befestigungslöchern bzw. Ösen eignet.

Die Abmessungen der erfindungsgemässen Beutel hängen selbstverständlich von der Menge an Schädlingsbekämpfungsmitteln ab, die diese aufnehmen sollen. Selbstverständlich ist es erforderlich, dass die erfindungsgemässen Beutel trotz der Volumenvergrösserung des Schädlingsbekämpfungsmittels, mit welcher bei der Ausgasung gerechnet werden muss, intakt bleiben.

Die vorliegende Erfindung wird durch die beiliegenden Zeichnungen näher erläutert, und zwar zeigt:

Figur 1 eine Aufsicht auf eine Lage des Vliesstoffes zur Herstellung eines erfindungsgemässen Einzelbeutels;

Figur 2 eine Aufsicht auf eine Lage eines Vliesstoffes zur Herstellung eines erfindungsgemässen Doppel- oder Mehrfachbeutels;

Figur 3 einen Querschnitt entlang der Linie III/III in Figur 2 in einem anderen Massstab;

Figur 4 eine Aufsicht auf eine Vliesstofflage zur Herstellung einer anderen Ausführungsform des erfindungsgemässen Beutels;

Figur 5 einen Querschnitt durch einen erfindungsgemässen Beutel, welcher sich in einer Verpackungsumhüllung befindet;

Figur 6 einen Querschnitt durch einen erfindungsgemässen Mehrfachbeutel;

Figur 7 eine Seitenansicht eines erfindungsgemässen Mehrfachbeutels, wie er gerade einem Behälter entnommen wird;

Figur 8 eine Aufsicht auf einen Blechbehälter (bei entferntem Deckel), der eine andere Ausführungsform der erfindungsgemässen Mehrfachbeutel enthält.

Obwohl zweifellos die bisherige detaillierte Beschreibung völlig ausreichend sein sollte, um ein leichtes Verstehen der Erfindung zu gewährleisten, wird an Hand der folgenden Figurenbeschreibung eine weitere Erläuterung der Erfindung gegeben. Die nun folgende Beschreibung soll in Verbindung mit den bereits vorstehend gemachten Ausführungen gesehen werden.

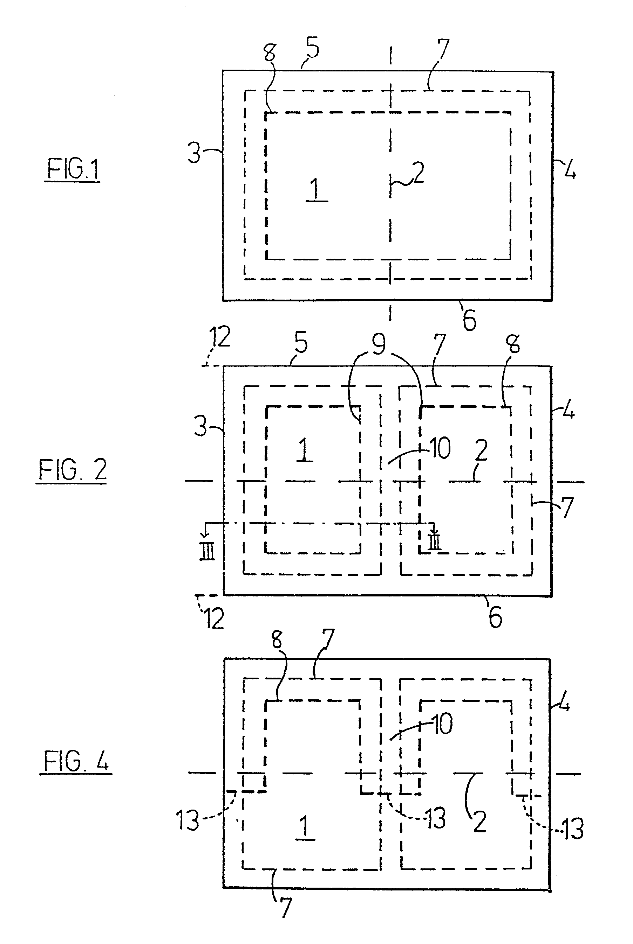

Bezugnehmend auf Figur 1 sei erläutert, dass mit 1 eine Lage eines Wasserdampf-durchlässigen, praktisch wasserfreien Vliesstoffes bezeichnet wird, wobei der Vliesstoff mindestens ein faserbildendes Material mit einem Schmelz- bzw. Erweichungspunkt über 165° C und gegebenenfalls ein zweites Material mit thermoplastischen Eigenschaften mit einem Schmelz- bzw. Erweichungspunkt unter 145° C umfasst. Die Lage zeigt die kurzen Seiten 3 und 4 (welche beispielsweise 7 bis 15 cm betragen können) und die langen Seiten 5 und 6 (welche z.B. 10 bis 20 cm ausmachen). Zur Bildung des erfindungsgemässen Beutels wird die Lage um die Mittellinie 2 derart gefaltet, dass die kurzen Seiten 3 und 4 aufeinander zu liegen kommen. Unter Verwendung von Hitze oder Ultraschall werden nun Schweissnähte in an sich bekannter Weise zwischen den gestrichelten Linien 7 und den Rändern (Seiten 3, 4, 5 und 6 der Lage) erzeugt, wobei jedoch eine Seite offen bleibt. Es wird eine abgemessene Menge an Schädlingsbekämpfungsmittel (beispielsweise zwischen 15 und 50 g, insbesondere 34 g) an der offenen Seite eingefüllt, worauf diese in gleicher Weise durch Hitze oder Ultraschall versiegelt wird. Selbstverständlich ist es nicht notwendig, an der mit 2 bezeichneten Seite eine Schweissnaht vorzusehen, wenn die Lage an dieser Stelle gefaltet ist, jedoch kann dieses wahlweise erfolgen. Die Anwendung des zweiten Materials oder des weiteren thermoplastischen Materials kann auf die Randzone zwischen der Linie 8 und über die Linie 7 hinaus, entlang der eine Schweissnaht vorgesehen ist, begrenzt sein. Die Schweissnaht kann beispielsweise eine Breite von 5 bis 10 mm, z.B. 8 mm aufweisen. Diese Begrenzung kann unter Verwendung einer Maske oder durch an sich bekannte Aufdruckverfahren (z.B. Siebdruck) zur Aufbringung der entsprechenden Komponente erfolgen. Wenn die betreffende Komponente dazu dienen soll, die Schweissnähte zu ermöglichen oder zu verbessern und wenn weiter ein geringerer Auftrag derselben Komponente dazu dienen soll, die Porengrösse des Vliesstoffes zu verändern, ist es selbstverständlich möglich, eine geringere Menge der genannten Komponente innerhalb der Linie 8 und einen stärkeren Auftrag selektiv zwischen der Linie 8 und den Rändern 3, 4, 5 und 6 vorzusehen.

Selbstverständlich ist es auch möglich, erfindungsgemässe Beutel der doppelten Grösse dadurch zu bilden, dass eine zweite Lage des Vliesstoffes, welche beispielsweise mit derjenigen von Figur 1 identisch ist, einander zugewandt auf die Lage 1 der Figur 1 gelegt wird. In diesem Fall werden die vier Seiten 3, 4, 5 und 6 rundum durch eine Schweissnaht verbunden, wobei zunächst eine Seite bis zur Einfüllung des Schädlingsbekämpfungsmittels offen gelassen wird.

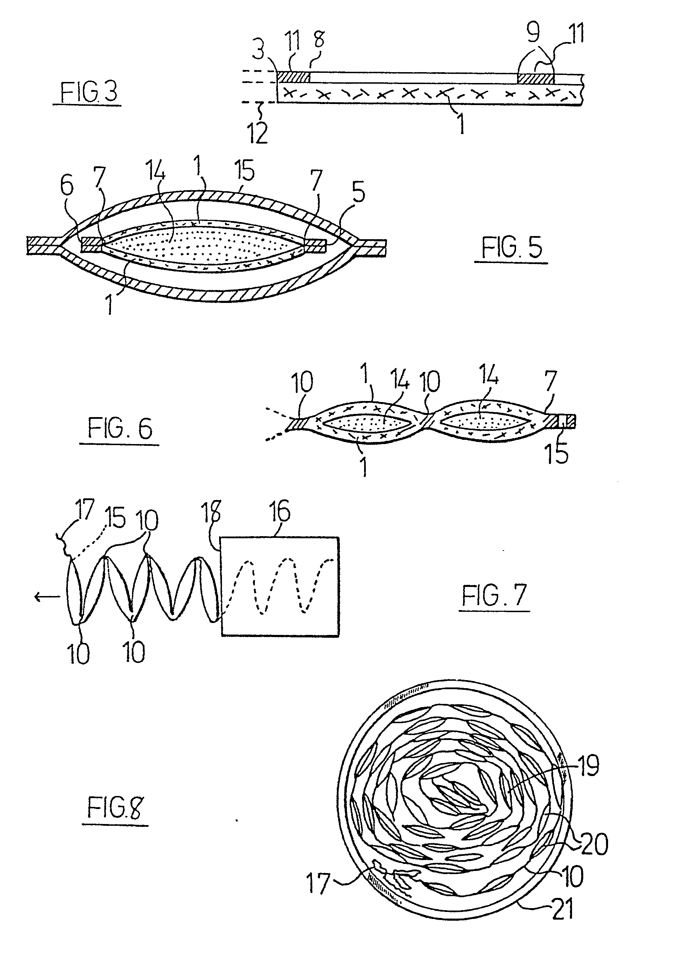

In den Figuren 2 und 3 werden dieselben Bezugszeichen wie in Figur 1 zur Bezeichnung derselben Teile verwendet. Im Gegensatz zur Ausführungsform nach Figur 1 wird die Vliesstofflage 1 zu einem Doppelbeutel oder Mehrfachbeutel verarbeitet, welche jeweils durch die Schweissnähte 10 verbunden sind, wobei dieselben Ausführungen, die oben hinsichtlich der Randzone gemacht worden sind, auch hier zutreffen (Begrenzung durch Linien 9 bzw. 3 und 8). Die gestrichelte Linie 2 in Figur 2 soll andeuten, dass sich die dargestellte Anordnung beliebig oft wiederholen kann, wobei die entsprechende Anzahl von nebeneinanderliegenden Beuteln erhalten wird. Durch Falten der Lage 1 bzw. Bahn entlang der Linie 2 werden jeweils Beutel der halben Grösse hergestellt, jedoch kann — wie bereits im Zusammenhang mit Figur 1 erläutert — auch unter Verwendung einer entsprechenden zweiten Lage oder Bahn des Vliesstoffes, welche auf die erste Bahn bzw. Lage gelegt wird und durch Schweissnähte mit der ersten verbunden wird, die Herstellung von erfindungsgemässen Beuteln erfolgen.

Wie in Figur 4 gezeigt, wo bei der Beutelherstellung die Lage bzw. Bahn um die Linie 2 gefaltet wird, kann auch die Beschichtung mit dem zweiten Material und/oder zusätzlichem thermoplastischen Material nur auf einer Seite der Faltlinie 2 erfolgen.

Es ist selbstverständlich, dass die Erläuterungen zu den Figuren 1 bis 4, welche die Herstellung von Einzel- oder Mehrfachbeuteln aus entsprechenden Stücken oder Lagen des Vliesstoffes betreffen, auch für die Verarbeitung von Endlosbahnen des Vliesstoffes, beispielsweise in entsprechenden maschinellen Vorrichtungen, zutreffend sind.

Figur 5 zeigt einen Einzelbeutel, welcher nach Figur 1 hergestellt worden ist und aus zwei Vliesstofflagen 1 besteht, wobei diese in den Bereichen 5/7 und 6/7 versiegelt sind und der Beutel mit Schädlingsbekämpfungsmitteln 14 in körnchenförmiger oder Pulverform gefüllt ist. Einer oder mehrere dieser Beutel werden von einer luftdichten, genügend starken Umhüllung 15, beispielsweise einer wärmeversiegelten, Aluminium-beschichteten Folie aufgenommen. Diese Umhüllung schützt die Beutel und das Schädlingsbekämpfungsmittel 14 insbesondere gegenüber der atmosphärischen Feuchtigkeit. Unmittelbar vor der Verwendung wird die Umhüllung 15 ohne Beschädigung der erfindungsgemässen Beutel aufgerissen oder aufgeschnitten. Die Beutel werden entfernt und möglichst schnell an dem zu begasenden Anwendungsort in bekannter Weise ausgelegt, wobei atmosphärische Feuchtigkeit durch den Vliesstoff 1 zu dem Schädlingsbekämpfungsmittel gelangen kann und eine Hydrolyse unter Gasentwicklung (Phosphin) in Gang setzt. Phosphin wird durch die Poren des Vliesstoffes 1 an die Umgebung abgegeben, so dass die Begasungswirkung eintreten kann. Die Hydrolyse ist normalerweise nach einigen Tagen beendet, wonach das Schädlingsbekämpfungsmittel aufgrund der Hydrolyse in Form eines lockeren Pulvers vorliegt, welches in dem bzw. den Beutel(n) eingeschlossen ist. Die Beutel verhindern somit eine Verunreinigung der Vorräte bzw. der Umgebung mit dem Pulver. Das Pulver hat ein deutlich kleineres Schüttgewicht als das ursprünglich eingesetzte Schädlingsbekämpfungsmittel.

Figur 6 zeigt einen Teil einer Anordnung mit einigen oder vielen erfindungsgemässen Beuteln. Diese kann beispielsweise eine Länge von 3 bis 10 m, z.B. 5 m, für 100 integrierte Beutel aufweisen. Die Breite kann beispielsweise 15 bis 32 cm betragen, wobei grössere Werte insbesondere dann erreicht werden, wenn sich zwei integrierte Beutel auf der bandförmigen Anordnung quer zur Längsrichtung nebeneinander befinden. Sofern an der Stelle, wo diese beiden Beutel zusammenstossen, eine doppelte Längsschweissnaht vorgesehen ist, können diese bei Bedarf auseinandergeschnitten werden. Die Flächen 10 stellen in der Zeichnung die Schweissnähte dar, wobei die äusserste Schweissnaht des ersten Beutels mit einem Loch 15 vorgesehen ist, durch das beispielsweise eine Schnur 17 hindurchgezogen werden kann. Gegebenenfalls kann dieses Loch 15 eine Metallöse aufnehmen.

In Figur 7 ist eine Anordnung mit einer grösseren Anzahl von erfindungsgemässen Beuteln dargestellt, die in Zickzack-Form gefaltet in einem gasdichten Behälter 16 verpackt worden waren. Die Zickzack-Form wird durch eine Faltung der Schweissnähte 10 zwischen den einzelnen Beuteln gebildet. Die äusserste Schweissnaht an einem Ende der Anordnung trägt ein Loch 15, durch das eine Befestigungsschnur 17 gezogen ist. In der Figur wurde der Behälter 16 an einer Seite (18) geöffnet und die Anordnung in Richtung des Pfeiles aus diesem teilweise herausgezogen.

Schliesslich zeigt Figur 8 eine andere Ausführungsform einer Anordnung 19 mit einer Vielzahl von erfindungsgemässen Beuteln 20, die über die Schweissnaht 10 zu einer langen bandartigen Anordnung miteinander verbunden sind. Diese ist aufgerollt und in einer gasdichten Blechbüchse 21 verpackt. An einem Ende der Anordnung ist wiederum eine Schnur 17 angebracht.

Der Vorzug des erfindungsgemässen Beutels liegt darin, dass er praktisch völlig gasdurchlässig ist, dennoch aber die staubförmigen Rückstände eines zerfallenen Schädlingsbekämpfungsmittels zurückhält. Ein erfindungsgemässer Beutel, der Schädlingsbekämpfungsmittel aufnehmen kann, besitzt darüber hinaus eine verbesserte Haltbarkeit und einen äusserst geringen Feuchtigkeitsgehalt. Dadurch wird eine vorzeitige unerwünschte Gasentwicklung bei der Herstellung verhindert. Ferner kann er durch problemlose Verschweissung unter Einfluss von Wärme bzw. Ultraschall in handelsüblichen Verschweissungs- und/oder Verpakkungsvorrichtungen hergestellt bzw. gefüllt werden. Die Schweissnähte besitzen eine genügend grosse Dichtigkeit und Festigkeit, so dass weder bei deren Bildung noch während des bestimmungsgemässen Verbrauchs der erfindungsgemässen Beutel die Gefahr besteht, dass das eingesiegelte Schädlingsbekämpfungsmittel bzw. dessen Rückstände austreten. Sie können also als absolut sicher und zuverlässig angesehen werden.

## Patentansprüche

1. Beutel für Schädlingsbekämpfungsmittel, zumindest teilweise bestehend aus einem Gas- und Wasserdampf-durchlässigen, wasserfreien Vliesstoff, dadurch gekennzeichnet, dass der Vliesstoff ein Mehrkomponentenvliesstoff ist, welcher mindestens ein faserbildendes Material mit einem Schmelz- bzw. Erweichungspunkt über 165° C und ein zweites Material mit thermoplastischen Eigenschaften und einem Schmelz- bzw. Erweichungspunkt unter 145° C umfasst.

2. Beutel nach Anspruch 1, dadurch gekennzeichnet, dass das faserbildende Material einen Schmelz- bzw. Erweichungspunkt zwischen 180 und 235° C aufweist.

3. Beutel nach mindestens einem der Ansprüche 1 bis 2, dadurch gekennzeichnet, dass das zweite Material einen Schmelz- bzw. Erweichungspunkt zwischen 80 und 120° C aufweist.

4. Beutel nach mindestens einem der Ansprüche 1 bis 3, dadurch gekennzeichnet, dass das zweite Material ebenfalls in Form von Fasern vorliegt.

5. Beutel nach mindestens einem der Ansprüche 1 bis 4, dadurch gekennzeichnet, dass das erste faserbildende Material ein Polyamid, Polyester, Polyacrylharz, Polypropylen mit relativ hohem Erweichungspunkt, Glasfasern oder eine Kombination von diesen umfasst.

6. Beutel nach mindestens einem der Ansprüche 1 bis 5, dadurch gekennzeichnet, dass die Faserlänge des ersten faserbildenden Materials 5 bis 20 mm beträgt.

7. Beutel nach mindestens einem der Ansprüche 1 bis 6, dadurch gekennzeichnet, dass das zweite Material Polyäthylen, Copolymere aus Äthylen, Propylen oder Butylen und Vinylacetat bzw. Polypropylen mit relativ niedrigem Erweichungspunkt umfasst.

8. Beutel nach mindestens einem der Ansprüche 4 bis 7, dadurch gekennzeichnet, dass die Faserlänge des zweiten Materials 0,5 bis 1 mm beträgt.

9. Beutel nach mindestens einem der Ansprüche 1 bis 8, dadurch gekennzeichnet, dass die Anteile des ersten faserbildenden Materials und des zweiten Materials 50:50 bis 70:30 Gew.-% betragen.

10. Beutel nach mindestens einem der Ansprüche 1 bis 9, dadurch gekennzeichnet, dass der Vliesstoff durch Verwendung eines Bindemittels verfestigt worden ist.

11. Beutel nach Anspruch 10, dadurch gekennzeichnet, dass das Bindemittel ein Polyacrylsäureester, Copolymer aus Äthylen, Propylen oder Butylen und Vinylacetat, Polyvinylchlorid oder Polyvinylacetat, vorzugsweise angewendet in Form einer Dispersion, oder ein Latex aus natürlichem oder künstlichem Kautschuk ist.

12. Beutel nach mindestens einem der Ansprüche 1 bis 11, dadurch gekennzeichnet, dass auf den Vliesstoff eine Beschichtung aus thermoplastischem Material, vorzugsweise Polyäthylen oder einem Äthylen/Vinylacetat-Copolymer, zumindest in den Bereichen, wo eine Verschweissungsnaht vorgesehen ist, aufgebracht worden ist.

13. Beutel nach Anspruch 12, dadurch gekennzeichnet, dass die Beschichtungsmenge zwischen 15 und 50 g/m², vorzugsweise zwischen 20 und 30 g/m², liegt.

14. Beutel nach mindestens einem der Ansprüche 1 bis 3, 5 und 6, dadurch gekennzeichnet, dass der Mehrkomponentenvliesstoff Fasern aus mindestens einem der faserbildenden Materialien umfasst, auf welchen zumindest teilweise das zweite Material, vorzugsweise Polyäthylen oder Äthylen/Vinylacetat-Copolymer, aufgebracht worden ist.

15. Beutel nach Anspruch 14, dadurch gekennzeichnet, dass das zweite Material durch Besinterung bzw. Aufdrucken oder Imprägnieren von Lösungen oder Emulsionen des zweiten Materials aufgebracht worden ist.

16. Beutel nach mindestens einem der Ansprüche 14 bis 15, dadurch gekennzeichnet, dass die beschichteten Fasern lediglich an den für eine Schweissnaht vorgesehenen Stellen des Vliesstoffes, und zwar vorzugsweise in einer Breite von mindestens 1 cm, bevorzugt 1 bis 3 cm, insbesondere 1 bis 2 cm, vorliegen.

17. Beutel nach Anspruch 1, dadurch gekennzeichnet, dass der Mehrkomponentenvliesstoff zumindest teilweise aus Kern-Mantel-Bikomponentenfasern besteht, wobei der Kern aus faserbildendem Material und der Mantel aus dem zweiten Material gebildet wird.

18. Beutel nach Anspruch 17, dadurch gekennzeichnet, dass der Kern aus Polyamid 6.6 und der Mantel aus Polyamid 6 besteht.

19. Beutel nach mindestens einem der Ansprüche 1 bis 18, dadurch gekennzeichnet, dass der Titer der Fäden des Vliesstoffes zwischen 1 und 20 dtex, vorzugsweise zwischen 1,5 und 3, insbesondere zwischen 1,7 und 2 dtex liegt.

20. Beutel nach mindestens einem der Ansprüche 1 bis 19, dadurch gekennzeichnet, dass der Vliesstoff ein Flächengewicht zwischen 50 und 120 g/m² besitzt.

21. Beutel nach mindestens einem der Ansprüche 1 bis 20, dadurch gekennzeichnet, dass er durch Verschweissen von Vliesstoffbahnen durch Wärme und/oder Ultraschall, gegebenenfalls nach Faltung an einer Seite, hergestellt worden ist.

22. Beutel nach mindestens einem der Ansprüche 1 bis 21, dadurch gekennzeichnet, dass er als Schlauchbeutel oder Siegelrandbeutel ausgebildet ist.

23. Beutel nach mindestens einem der Ansprüche 1 bis 22, dadurch gekennzeichnet, dass er ein Element eines Flächengebildes darstellt.

24. Beutel nach Anspruch 23, dadurch gekennzeichnet, dass es sich um ein flexibles Flächengebilde, vorzugsweise in Form einer ausrollbaren Bahn oder in Zickzack-Form gefaltet, handelt.

25. Beutel nach Anspruch 24, dadurch gekennzeichnet, dass die Bahn aus dem gleichen Material wie der Beutel besteht und gegebenenfalls eine oder beide Seiten von diesem bildet.

26. Beutel nach mindestens einem der Ansprüche 1 bis 25, dadurch gekennzeichnet, dass die nach dem Füllen verbleibende Öffnung durch Schweissen verschlossen worden ist.

27. Beutel nach mindestens einem der Ansprüche 1 bis 26, dadurch gekennzeichnet, dass es sich bei dem Vliesstoff um einen vorzugsweise hydrodynamisch hergestellten Wirrvliesstoff handelt.

28. Beutel nach mindestens einem der Ansprüche 1 bis 27, dadurch gekennzeichnet, dass sie in an sich bekannter Weise ganz oder teilweise gefärbt und/oder bedruckt sind.

29. Beutel nach mindestens einem der Ansprüche 1 bis 28, dadurch gekennzeichnet, dass die Fasern, der Vliesstoff und/oder der fertige Beutel mit einem hydrophobierenden Mittel behandelt worden sind.

30. Verwendung des Beutels nach mindestens einem der Ansprüche 1 bis 29 zur Aufnahme ausgasungsfähiger Schädlingsbekämpfungsmittel, insbesondere einer Phosphin entwickelnden Zusammensetzung auf der Basis von Erdalkali- und/oder Erdmetallphosphiden.

## Revendications

1. Sachet pour pesticides constitué au moins en partie par un non-tissé anhydre, perméable aux gaz et à la vapeur d'eau, caractérisé en ce que le non-tissé est un non-tissé multicomposant qui comprend au moins une matière fibreuse avec un point de fusion, respectivement de ramollissement, supérieur à 165° C et une seconde matière possédant des propriétés thermoplastiques et un point de fusion, respectivement de ramollissement, inférieur à 145° C.

2. Sachet selon la revendication 1, caractérisé en ce que la matière fibreuse présente un point de fusion, respectivement de ramollissement, compris entre 180 et 235° C.

3. Sachet selon au moins une des revendications 1 à 2, caractérisé en ce que la seconde matière présente un point de fusion, respectivement de ramollissement, compris entre 80 et 120° C.

4. Sachet selon au moins une des revendications 1 à 3, caractérisé en ce que la seconde matière se présente également sous forme de fibres.

5. Sachet selon au moins une des revendications 1 à 4, caractérisé en ce que la première matière fibreuse comprend un polyamide, un polyester, une résine polyacrylique, du polypropylène avec un point de ramollissement relativement élevé, des fibres de verre ou une combinaison de ceux-ci.

6. Sachet selon au moins une des revendications 1 à 5, caractérisé en ce que la longueur de fibre de la première matière fibreuse est de 5 à 20 mm.

7. Sachet selon au moins une des revendications 1 à 6, caractérisé en ce que la seconde matière comprend du polyéthylène, des copolymères d'éthylène, de propylène ou de butylène et d'acétate de vinyle, ou du polypropylène avec un point de ramollissement relativement bas.

8. Sachet selon au moins une des revendications 4 à 7, caractérisé en ce que la longueur de fibre de la seconde matière s'élève de 0,5 à 1 mm.

9. Sachet selon au moins une des revendications 1 à 8, caractérisé en ce que les proportions de la première matière fibreuse et de la seconde matière vont de 50:50 à 70:30% en poids.

10. Sachet selon au moins une des revendications 1 à 9, caractérisé en ce que la nappe a été consolidée par emploi d'un liant.

11. Sachet selon la revendication 10, caractérisé en ce que le liant est un ester polyacrylique, un copolymère d'éthylène, de propylène ou de butylène et d'acétate de vinyle, du poly(chlorure de vinyle) ou du poly(acétate de vinyle), avantageusement mis en œuvre sous la forme d'une dispersion, ou un latex de caoutchouc naturel ou synthétique.

12. Sachet selon au moins une des revendications 1 à 11, caractérisé en ce qu'un revêtement en une matière thermoplastique, de préférence du polyéthylène ou un copolymère d'éthylène/acétate de vinyle, a été appliqué sur la nappe, du moins aux endroits où est prévu un cordon de soudure.

13. Sachet selon la revendication 12, caractérisé en ce que la quantité déposée se situe entre 15 et 50 g/m², de préférence entre 20 et 30 g/m².

14. Sachet selon au moins une des revendications 1 à 3, 5 et 6, caractérisé en ce que le non-tissé multicomposant comprend des fibres en au moins une des matières fibreuses sur laquelle a été appliquée au moins en partie la seconde matière, de préférence du polyéthylène ou un copolymère d'éthylène/acétate de vinyle.

15. Sachet selon la revendication 14, caractérisé en ce que la seconde matière a été appliquée par saupoudrage, respectivement par impression ou imprégnation de solutions ou d'émulsions de la seconde matière.

16. Sachet selon au moins une des revendications 14 à 15, caractérisé en ce que les fibres revêtues se trouvent uniquement aux endroits de la nappe prévue pour le cordon de soudure, avantageusement sur une largeur d'au moins 1 cm, de préférence de 1 à 5 cm, en particulier de 1 à 2 cm.

17. Sachet selon la revendication 1, caractérisé en ce que le non-tissé multicomposant est au moins partiellement composé de fibres bicomposantes à âme-enveloppe, l'âme étant constituée par la matière fibreuse et l'enveloppe par la seconde matière.

18. Sachet selon la revendication 17, caractérisé en ce que l'âme est constituée par du polyamide 6,6 et l'enveloppe par du polyamide 6.

19. Sachet selon au moins une des revendications 1 à 18, caractérisé en ce que le titre des fils de la nappe se situe entre 1 et 20 dtex, de préférence entre 1,5 et 3, en particulier entre 1,7 et 2 dtex.

20. Sachet selon au moins une des revendications 1 à 19, caractérisé en ce que la nappe possède une masse par unité de surface comprise entre 50 et 120 g/m².

21. Sachet selon au moins une des revendications 1 à 20, caractérisé en ce qu'il a été réalisé par soudage de bandes de non-tissés par la chaleur et/ou par ultrasons, éventuellement après pliage sur un côté.

22. Sachet selon au moins une des revendications 1 à 21, caractérisé en ce qu'il a la forme d'un sachet tubulaire ou d'un sachet à bords scellés.

23. Sachet selon au moins une des revendications 1 à 22, caractérisé en ce qu'il représente un élément d'une structure en nappe.

24. Sachet selon la revendication 23, caractérisé en ce qu'il s'agit d'une structure en nappe flexible, de préférence sous forme d'une bande déroulable ou pliée en zigzag.

25. Sachet selon la revendication 24, caractérisé en ce que la bande est composée de la même matière que le sachet et constitue éventuellement un ou deux côtés de celui-ci.

26. Sachet selon au moins une des revendications 1 à 25, caractérisé en ce que l'ouverture restant après le remplissage a été scellée par soudage.

27. Sachet selon au moins une des revendications 1 à 26, caractérisé en ce que, dans le cas du non-tissé, il s'agit d'un non-tissé enchevêtré réalisé de préférence par voie hydrodynamique.

28. Sachets selon au moins une des revendications 1 à 27, caractérisés en ce qu'ils sont en partie ou entièrement teints et/ou imprimés de façon connue en soi.

29. Sachet selon au moins une des revendications 1 à 28, caractérisé en ce que les fibres, la nappe et/ou le sachet fini ont été traités par un agent hydrofugeant.

30. Utilisation du sachet selon au moins une des revendications 1 à 29 pour recevoir des pesticides, capables de désinfecter par gaz, notamment une composition dégageant une phosphine à base de phosphures de métaux alcalino-terreux et/ou de métaux terreux.

## Claims

1. Bags for pest control agents, at least partially consisting of a gas and steam permeable anhydrous non-woven material, characterized in that said non-woven material is a multi-component non-woven material, comprising at least a fiber-forming material having a melting or softening point above 165° C and a second material having thermoplastic properties and a melting or softening point below 145° C.

2. Bags according to claim 1, characterized in that the fiber-forming material shows a melting or softening point between 180 and 235° C.

3. Bags according to at least one of claims 1 to 2, characterized in that the second material shows a melting or softening point between 80 and 120° C.

4. Bags according to at least one of claims 1 to 3, characterized in that the second material also is present in the form of fibers.

5. Bags according to at least one of claims 1 to 4, characterized in that the first fiber-forming material comprises a polyamide, polyester, polyacrylic resin, polypropylene having a relatively high softening point, glass fibers or a combination thereof.

6. Bags according to at least one of claims 1 to 5, characterized in that the fiber length of the first fiber-forming material is 5 to 20 mm.

7. Bags according to at least one of claims 1 to 6, characterized in that the second material comprises polyethylene, copolymers of ethylene, propylene or butylene and vinylacetate or polypropylene having a relatively low softening point.

8. Bags according to at least one of claims 4 to 7, characterized in that the fiber length of the second material is 0.5 to 1 mm.

9. Bags according to at least one of claims 1 to 8, characterized in that the portions of the first fiber-forming material and the second material are 50:50 to 70:30 weight%.

10. Bags according to at least one of claims 1 to 9, characterized in that the non-woven material has been compacted by the use of a binder.

11. Bags according to claim 10, characterized in that the binder is a polyacrylic acid ester, a copolymer of ethylene, propylene or butylene and vinylacetate, polyvinyl chloride or polyvinyl acetate, preferably used in the form of a dispersion, or a latex of natural or synthetic caoutchouc.

12. Bags according to at least one of claims 1 to 11, characterized in that a coating of a thermoplastic material, preferably polyethylene or an ethylene/vinylacetate copolymer, has been applied to the non-woven material, at least in those areas where a sealing seam is intended.

13. Bags according to claim 12, characterized in that the amount of the coating is between 15 and 50 g/m², preferably between 20 and 30 g/m².

14. Bags according to at least one of claims 1 to 3, 5 and 6, characterized in that the multi-component non-woven material comprises fibers of at least one of the fiber-forming materials, onto which the second material, preferably polyethylene or an ethylene/vinylacetate copolymer, has been applied at least partially.

15. Bags according to claim 14, characterized in that the second material has been applied by sintering or imprinting or impregnating of solutions or emulsions of the second material.

16. Bags according to at least one of claims 14 to 15, characterized in that the coated fibers are present only in those areas of the non-woven material which are intended for a sealing seam, namely preferably in a width of at least 1 cm, preferably 1 to 3 cm, especially 1 to 2 cm.

17. Bags according to claim 1, characterized in that the multi-component non-woven material at least partially consists of coated core-bicomponent fibers, the core being formed of the fiber-forming material and the coating being formed of the second material.

18. Bags according to claim 17, characterized in that the core consists of polyamide 6.6 and the coating of polyamide 6.

19. Bags according to at least one of claims 1 to 18, characterized in that the titre value of the filaments of the non-woven material is between 1 and 20 dtex, preferably between 1.5 and 3, especially between 1.7 and 2 dtex.

20. Bags according to at least one of claims 1 to 19, characterized in that the non-woven material has a weight per unit area between 50 and 120 g/m².

21. Bag according to at least one of claims 1 to 20, characterized in that it has been manufactured by sealing lengths of non-woven material by heat and/or ultrasonics, if desired after folding at one side.

22. Bag according to at least one of claims 1 to 21, characterized in that it is manufactured in the form of a tubular or a side-sealed bag.

23. Bag according to at least one of claims 1 to 22, characterized in that it represents an element of a flat structure.

24. Bag according to claim 23, characterized in that it is a flexible flat structure, preferably in the form of an elongated belt to be rolled up or folded up concertina-like.

25. Bag according to claim 24, characterized in that the belt consists of the same material as the bag, if desired one or both sides being formed thereof.

26. Bags according to at least one of claims 1 to 25, characterized in that the opening remaining after filling has been closed by sealing.

27. Bags according to at least one of claims 1 to 26, characterized in that the non-woven material is a tangled fiber web, preferably manufactured hydrodynamically.

28. Bags according to at least one of claims 1 to 27, characterized in that they have been wholly or partly dyed and/or printed in per se known manner.

29. Bags according to at least one of claims 1 to 28, characterized in that the fibers, the non-woven material and/or the finished bags have been treated with a hydrophobizing material.

30. The use of the bag according to at least one of claims 1 to 29 to contain pest control agents which may release gaseous substances, especially a phosphine releasing composition on the basis of earth alkaline and/or earth metal phosphides.

FIG.1

FIG. 2

FIG. 4

FIG.3

FIG.5

FIG.6

FIG.7

FIG.8